# FASCICULE DE BREVET EUROPEEN

(11) **EP 0 491 628 B1**
(45) Date de publication et mention de la délivrance du brevet: **29.09.1999**
(21) Numéro de dépôt: 91403446.7
(22) Date de dépôt: 18.12.1991
(51) Int. Cl.: C07K 14/00, A61K 47/48

(54) **Lipopeptides inducteurs des lymphocytes T-cytotoxiques et utilisation comme vaccins**
Lipopeptide, T-cytotoxische Lymphocyte aktivierend, und deren Verwendung als Vakzine
Lipopeptide inducing T-cytotoxic lymphocytes and their use as vaccines

(30) Priorité: 18.12.1990 FR 9015870
(43) Date de publication de la demande: 24.06.1992
(60) Demande divisionnaire: 99105773.8
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: Boutillon, Christophe, F-59800 Lille (FR); Martinon, Frédéric, F-92120 Montrouge (FR); Sergheraert, Christian, F-59190 Morbecque (FR); Magne, Rémy, F-78620 L'Etang La Ville (FR); Gras-Masse, Hélèna, F-59710 Merignies (FR); Gomard, Elisabeth, F-75017 Paris (FR); Tartar, André, F-62490 Vitry en Artois (FR); Levy, Jean-Paul, F-75013 Paris (FR)
(74) Mandataire: Phélip, Bruno

(56) Documents cités:
- EP-A- 0 093 851
- EP-A- 0 203 676
- EP-A- 0 306 912
- WO-A-89/10348
- VACCINE, vol. 7, no. 1, février 1989, pages 29-33, Butterworth & Co. (Publishers) Ltd, Guildford, Surrey, GB; K.-H. WIESMÜLLER et al.: "Novel low-molecular-weight synthetic vaccine against foot-and-mouth disease containing a potent B-cell and macrophage activator"
- CHEMICAL ABSTRACTS, vol. 106, no. 25, 22 juin 1987, page 264, résumé no. 209643p, Columbus, Ohio, US; J. MERCY et al.: "Circular dichroism studies on a synthetic peptide corresponding to the membrane-spanning region of vesicular stomatitis virus G protein and its fatty acyl derivative", & BIOCHIM. BIOPHYS. ACTA 1987, 911(2), 231-7
- CHEMICAL ABSTRACTS, vol. 106, no. 19, 11 mai 1987, page 516, résumé no. 154381u, Columbus, Ohio, US; E. WATARI et al.: "A synthetic peptide induces long-term protection from lethal infection with herpes simplex virus 2", & J. EXP. MED. 1987, 165(2), 459-70
- CHEMICAL ABSTRACTS, vol. 104, no. 21, 26 mai 1986, page 472, résumé no. 184455x, Columbus, Ohio, US; C.O. JACOB et al.: "Effect of carrier on the immunogenic capacity of synthetic cholera vaccine", & MOL. IMMUNOL. 1985, 22(12), 1333-9
- CHEMICAL ABSTRACTS, vol. 100, no. 17, 23 avril 1984, page 479, résumé no. 137063a, Columbus, Ohio, US; T.P. HOPP: "immunogenicity of a synthetic HBsAg peptide: enhancement by conjugation to a fatty acid carrier", & MOL. IMMUNOL. 1984, 21(1), 13-16

## Description

La présente invention a pour objet de nouveaux lipopeptides inducteurs des lymphocytes T cytotoxiques.

Elle a d'autre part pour objet l'utilisation de tels lipopeptides comme vaccins.

La plupart des vaccins utilisés induisent une réponse par l'intermédiaire des anticorps. Néanmoins , il a été montré que les lymphocytes cytotoxiques peuvent de manière efficace protéger des souris contre divers microorganismes pathogènes (Skehel et al, Proc. Natl. Acad. Sci. USA.1982, 79; 968 ; Lukacher al, Exp. Med. 1984 , 160: 814). Ceci a été aussi montré pour des lymphocytes T cytotoxiques humains dirigés contre des cytomégalovirus (Quinnan et al., N. Engl. J. Med. 1982, 307: 7, Rook et al , Am. J. Med. 1984, 76: 385). Cependant peu de choses sont connues concernant l'induction de l'immunité due aux lymphocytes.

Des auteurs ont tenté d'induire in vivo des lymphocytes T cytotoxiques (CTL) à l'aide de peptides dérivés de l'ovalbumine (Carbone et al. J. Exp. Med. 169: 603, Ishioka et al. 1989, J. Immunol. 143:1094). Ces auteurs ont obtenu des immunisations, mais ces résultats sont spécifiques des peptides dérivés de l'ovalbumine .

AICHELE et al. ( 1990) J. Exp. Med. 171: 1815) ont , quant à eux réussi à induire une réponse T cytolytique par des injections répétées in vivo d'un peptide synthétique en émulsion dans de l'adjuvant incomplet de Freund . Ces auteurs n'indiquent pas l'importance de l'adjuvant dans leurs conditions d'immunisation. Cependant, ils laissent supposer qu'un adjuvant est nécessaire à l'obtention d'une telle réponse .

La demande EP-203.676 a pour objet un vaccin destiné à induire une réponse par l'intermédiaire des cellules T , comprenant des conjugués peptide-acide gras. L'acide gras utilisé est l'acide palmitique. Cependant , ce vaccin comprend aussi de l'adjuvant de Freund .

A la connaissance du demandeur , seuls DERES et al. (Nature, Volume 342, 30 Novembre 1989) ont décrit l'utilisation d'un lipopeptide synthétique pour induire in vivo les lymphocytes T cytotoxiques (CTL) . Dans cet article , le fragment NP 147-158 d'une nucléoprotéine du virus influenza est couplé avec du tripalmitoyl-S-glycéryl cystéinyl-séryl-sérine(P3CSS). Il est montré que le conjugué peptide NP 147-158-lipide P3CSS induit une réponse CTL contre des cellules cibles infectées par le virus influenza, tandis que des souris immunisées avec seulement le peptide NP 147-158 ou avec le peptide Ser Ser-NP 147-158 ne génèrent pas de lymphocytes T cytotoxiques dirigés contre ce virus.

Il est aussi à noter que des lipopeptides ont déjà été utilisés pour induire des réactions immunologiques vis-à-vis d'antigènes précis , mais les réponses engendrées impliquent la synthèse d'anticorps, et non des réponses lymphocytaires T.

HOPP ( Molecular Immunology, 21, 13-16, 1984) a montré que l'on pouvait obtenir des anticorps dirigés contre un déterminant du virus de l'hépatite B en immunisant des lapins à l'aide d'une molécule constituée d'un peptide de 15 acides aminés correspondant au déterminant antigénique du virus de l'hépatite B et d'un résidu pseudolipidique, la dipalmitoyl lysine.

La demande EP-93.851 décrit des lipopeptides comprenant une séquence peptidique de 6 à 50 acides aminés liée à une partie lipophile telle qu'un acide gras palmitique, stéarique , béhénique, oléique ou mycolique . Il est mentionné que ces lipopeptides induisent la synthèse d'anticorps.

L'article de Wiesmüller et al (Vaccine, vol.7, n°1, 29-33, 1989) décrit l'utilisation d'un lipopeptide formé d'une partie de la séquence du virus FMDV ( VP₁) et du lipide P₃CSS pour induire la synthèse d'anticorps.

Le résumé de l'article de Jacob et al (Chemical Abstracts, vol.104, n°21, 472, abrégé 184.455 x, 1986) concerne l'induction de la synthèse d'anticorps par un lipopeptide formé de la toxine tétanique liée à un résidu dipalmityl.

Le résumé de l'article de Watari et al (Chemical Abstracts, vol. 106, p 516, résumé n° 154.381 u, 1987) est relatif à l'utilisation de peptides correspondant à la région N-terminale de la glycoprotéine D du virus HSV couplés à l'acide palmitique . Il est indiqué clairement dans cet article qu'il y a induction d'une réponse par l'intermédiaire des cellules T mais que cette réponse n'est pas due à des lymphocytes T cytotoxiques .

Deux autres documents concernent la synthèse et l'étude de la structure de lipopeptides .

La demande Internationale WO 89 10 348 a pour objet des lipopeptides dérivés d'acides gras tels que les acides aminoeicosanique, aminodécanoïque, aminotétradécanoïque, bromodécanoïque et bromododécanoïque.

Il est mentionné que ces composés peuvent être utilisés comme adjuvants et support pour vaccins, mais sans donner de moyens de mise en oeuvre.

Le résumé de l'article de Mercy et al (Chemical Abstracts, vol.106, n°25, 264, abrégé n° 209.643 p, 1987) concerne l'analyse structurale d'un lipopeptide formé d'un fragment d'une protéine du virus G et du lysine-palmitoyl.

Cette analyse de l'état de la technique montre donc qu'aucune technologie applicable à différents types de déterminants antigéniques n'a été mise au point permettant d'obtenir l'induction des lymphocytes T cytotoxiques, avec une réponse induite forte, et ne nécessitant pas d'administration d'adjuvant .

Le demandeur a montré de manière surprenante que l'on pouvait induire chez un organisme hôte une réponse des lymphocytes T cytotoxiques contre un antigène en immunisant ledit organisme avec un complexe lipopeptidique contenant un des déterminants de cet antigène .

De manière encore plus surprenante, le demandeur a montré que cette induction pouvait être obtenue pour un grand nombre de déterminants antigéniques de divers agents pathogènes.

La présente invention a donc pour objet l'utilisation d'un lipopeptide comprenant une partie peptidique ayant entre 10 et 40 et préférentiellement entre 10 et 20 acides aminés environ et comportant au moins un déterminant antigénique induisant spécifiquement des lymphocytes T cytotoxiques, ledit lipopeptide comprenant également une ou plusieurs chaînes dérivées de la N-ε-palmitoyl-lysine couplées sur des fonctions NH₂-α, ou NH₂-ε desdits acides aminés, pour la fabrication d'un médicament pour l'immunisation du coprs humain ou animal à l'encontre d'agents pathogènes.

Lesdits dérivés d'acides gras peuvent être notamment:
la N-ε-palmitoyl-lysine (L) de formule (II)suivante: 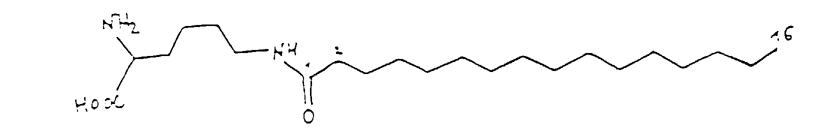
- ou son dérivé N-ε-palmitoyl-lysylamide de formule : 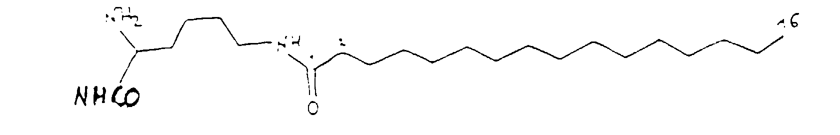
la N,N-diplamitoyl-lysine (L) de formule (III) suivante: 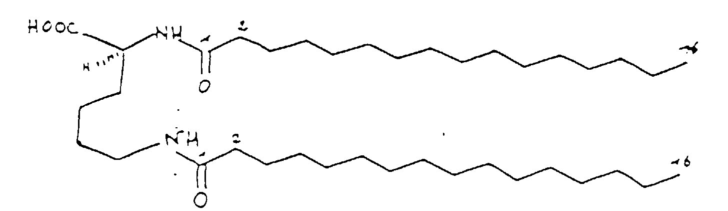
ou un de leurs dérivés.

La partie peptidique peut être un fragment de toute protéine issue d'agent pathogène présentant un déterminant antigénique.

De telles protéines peuvent notamment être les protéines du virus HIV1 ou HIV2, en particulier la protéine codée par le gène env. Dans ce cas, on peut de manière avantageuse utiliser les fragments 312-327 ou 302-336 selon la séquence HIV₁-BRU ( Hyers, C.A.B. Rabson, S.F. Josephs, T.F. Smith and E. Wong-Staal (Eds.). 1989. Human retrovirus and AIDS. Los Alamos Laboratoy II:59) de cette protéine, pour former la molécule conjuguée lipopeptidique.

La présente invention a d'autre part pour objet des vaccins à l'encontre de virus ou de parasites contenant l'un des lipopeptides précédemment décrits , et en particulier des vaccins à l'encontre des maladies liées aux virus HIV, lesdits vaccins contenant avantageusement un fragment de la protéine produit du gène env.

La présente invention a de plus pour objet des compositions pharmaceutiques contenant une quantité efficace d'au moins un des composés précédemment décrits en association avec un ou plusieurs diluants ou adjuvants compatibles et pharmaceutiquement acceptables . Ces compositions sont en particulier destinées au traitement des maladies liées aux virus HIV par induction des lymphocytes T cytotoxiques .

La présente invention a encore pour objet des lipopeptides précédemment décrits et comprenant un fragment d'une protéine des virus HIV1 ou HIV2. Des agents pathogènes à l'encontre desquels le corps peut être immunisé peuvent être des virus ayant une dépense cytotoxique importante , en particulier les virus HIV1 et HIV2, et certains parasites.

Lesdits lipopeptides peuvent aussi être utilisés contre certains cancers afin d'induire une réponse CTL spécifique de certaines cellules tumorales.

Les lipopeptides objets de la présente invention peuvent être obtenus à partir des constituants protéiques et pseudolipidiques par des méthodes connues de l'homme du métier , en particulier soit par couplage des acides aminés composant la partie peptidique avec le pseudo-lipide immobilisé sur une résine, c'est-à-dire par synthèse en phase solide, soit par couplage du pseudo-lipide sur un peptide immobilisé en phase solide.

Il est à remarquer que les lipopeptides selon l'invention, présentent l'avantage notable de pouvoir être adaptés à l'induction de lymphocytes T cytotoxiques dirigés contre tout type de déterminant antigénique de tout agent pathogène.

A titre subsidiaire la présente invention concerne aussi les intermédiaires de synthèse suivants:
l'acidee 2-tertiobutyloxycarbonylamino hexadécanoïque (D,L) de formule (VIII) suivante : 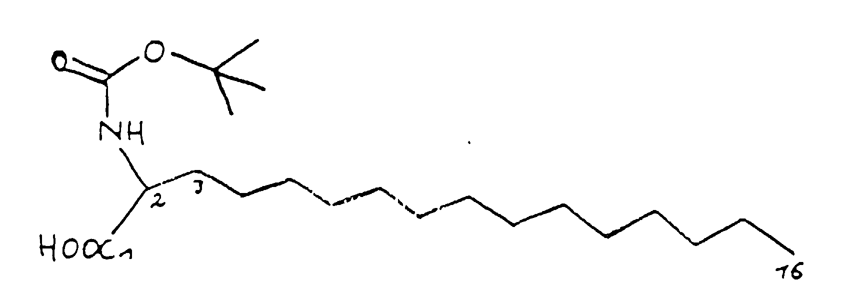
la N-α-terbutyloxycarbonyl ε-palmitoyl-lysine (L) de formule (IX) suivante : 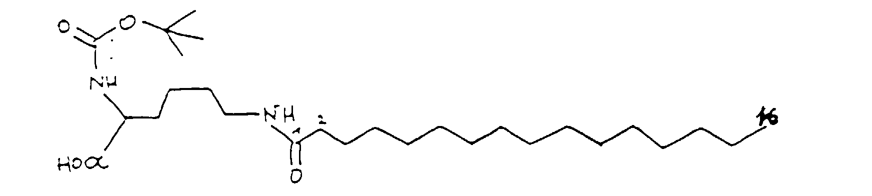
la N-α-fluorénylméthyloxycarbonyl ε-palmitoyl-lysine (L) de formule (X) suivante : 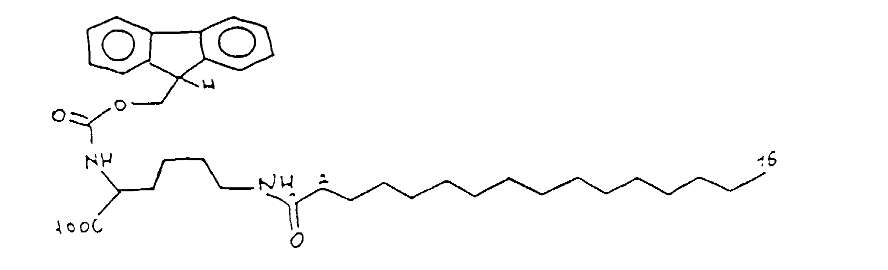
la N-α-tertiobutyloxycarbonyl ε-[(cholest-5-ényl-3-oxy)-acétyl] -lysine (L) de formule (XI) suivante : 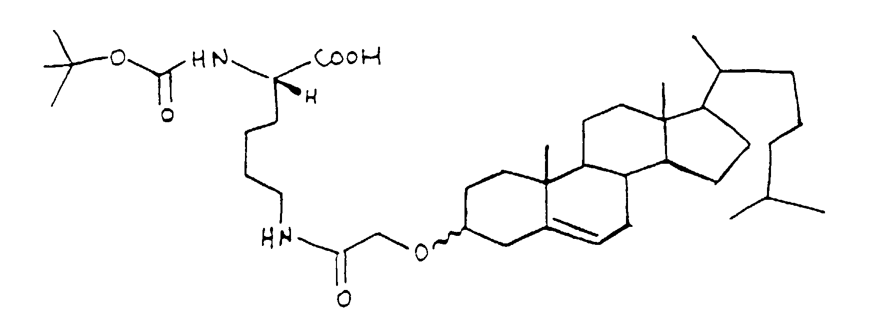
ou la N-α-fluorénylméthyloxycarbonyl ε-[(cholest-5-ényl-3-oxy)-acétyl] lysine (L) de formule (XII) suivante: 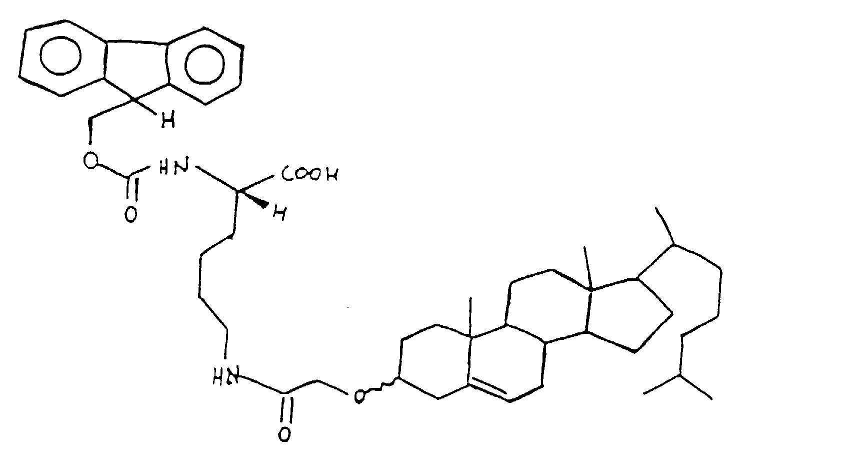
ou un de leurs dérivés.

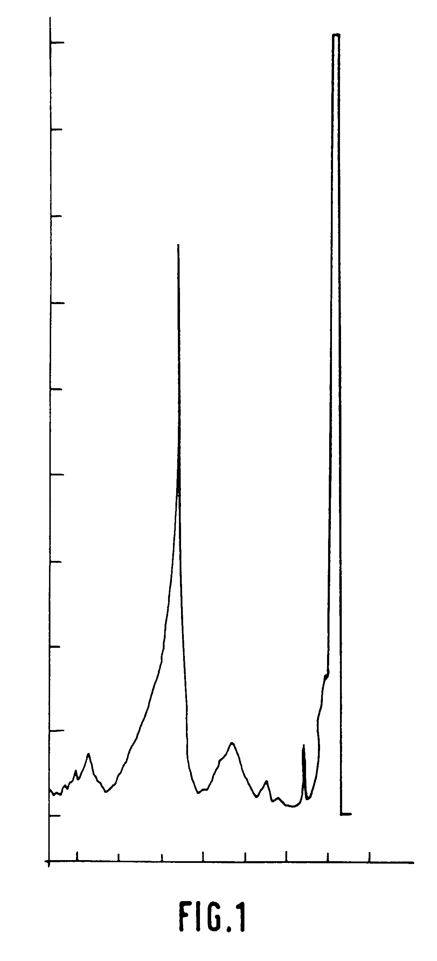

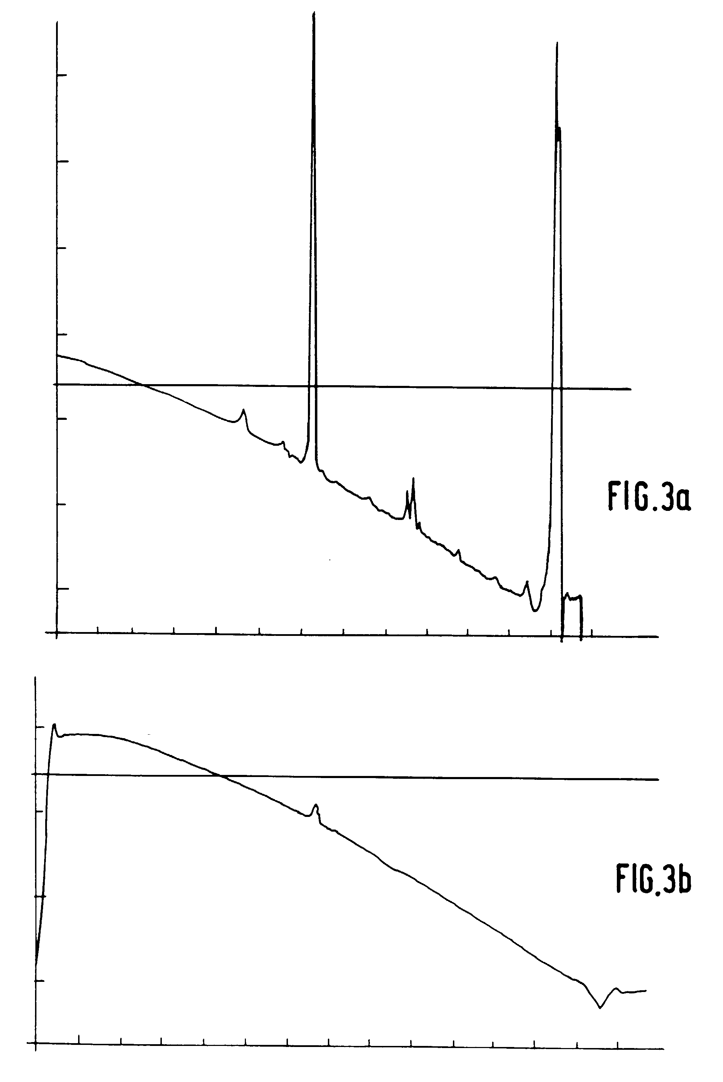

La présente invention est illustrée sans être aucunement limitée par les exemples d'application suivants dans lesquels les figures 1 et 3 représentent les spectres en HPLC en phase inverse du lipopeptide V3GP120, 312-327 succinyl respectivement lors des électrophorèses préparatives et analytiques .

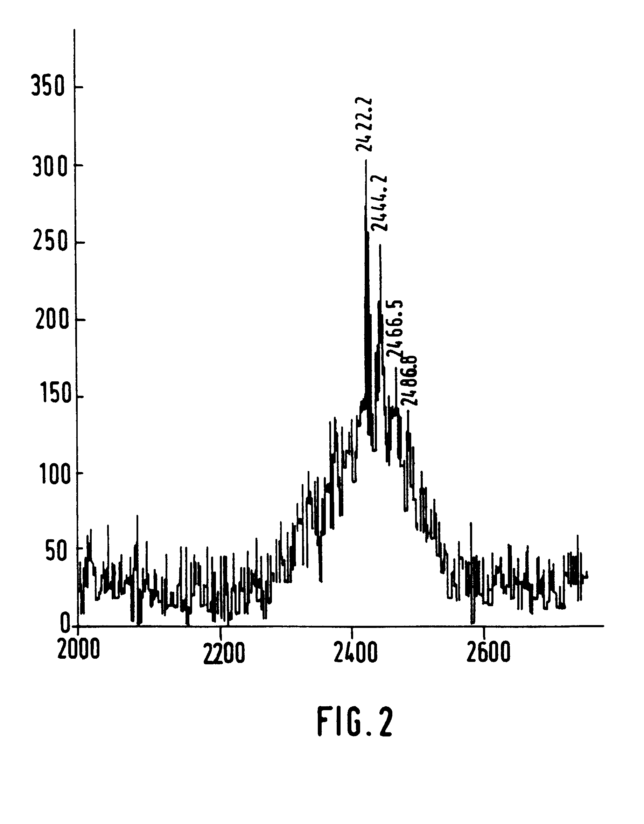

La figure 2 représente quant à elle son spectre de masse .

### EXEMPLE 1.

### Synthèses des résidus pseudo-lipidiques ( ou molécules hydrophobes ).

### 1. Synthèse de l'acide t-butyloxycarbonyl amino-2-hexadécanoïque .

### 1.1 Synthèse de l'acide amino-2 héxadécanoïque.

Formule brute : C₁₆H₃₃NO₂
Masse moléculaire : 271,44
Mode opératoire :

Dans un autoclave préalablement nettoyé à l'acide nitrique 50% et à l'acide phosphorique 10%, on introduit 0,0298 mole d'acide 2-bromohexadécanoïque (10g) et 100 ml de NH₄OH en solution à 28%. Après agitation, l'autoclave est chauffé à 60°C pendant 15 heures . Le dérivé aminé en formation précipite dans le milieu réactionnel. Après refroidissement , l'autoclave est lavé , l'eau diffusée et l'éthanol éliminé. Le mélange réactionnel est filtré sur verre fritté de porosité 4. La différence de solubilité des différents produits en milieu éthanol permet d'éliminer les traces d'acide 2-bromohexadécanoïque par rinçages .

Quantité de précipité obtenu : 4,37 g ; rendement de 54%.

Remarque : la non solubilité du dérivé aminé a été vérifiée dans de nombreux solvants ( eau, éthanol, acide acétique à froid, acide formique 70%, acétate d'éthyle, toluène, toluène/acétonitrile, acétate d'éthyle/acétonitrile). Parmi tous les essais de solubilisation testés, seule l'action d'un détergent spécifique ( le triméthylbenzylammonium hydroxyde) ou de l'acide acétique bouillant a dissous le dérivé aminé.

Le précipité séché est repris par 150 ml d'acide acétique chauffé à reflux jusqu'à obtention d'une solution limpide jaunâtre . Les pigments colorés sont absorbés sur charbon végétal . Après filtration sur papier filtre plissé, le dérivé aminé purifié est obtenu par cristallisation à partir de l'éluat. Les cristaux blancs obtenus sont récupérés sur verre fritté de porosité 4, lavés à l'acide acétique froid avant d'être séchés en dessicateur sur P₂O₅.

Rendement : 46 % ( le produit étant sous forme acétate on considère un PM de 330,48).

Le rendement de purification équivaut à 85%.

### 1.2. Synthèse de l'acide T-butyloxycarbonyllamino-2-hexadécanoïque.

- Formule brute : C₂₁H₄₁NO₄
- Masse moléculaire : 371,557.
- Mode opératoire :

### Mise en solution de l'acide amino-2 hexadécanoïque .

A 5 mmoles d'acide aminé sous forme acétate (1.655g) sont ajoutés 1.1 équivalent de "Triton" (benzyltriméthylammoniumhydroxyde) en solution à 40% dans le méthanol (2.9 ml) ainsi que 100 ml de DMF. Le mélange réactionnel est laissé sous agitation à température ambiante jusqu'à mise en solution complète. Le DMF est alors évaporé à la pompe à palettes . Le résidu est séché en dessicateur sur P₂O₅.

### Protection de la fonction amine .

Le résidu sec est dissous dans un mélange constitué de 36 ml d'eau , 8 ml de KHCO₃ 1N et 30 ml d'alcool tertiobutylique. A cette solution, sont ajoutés 2,5 équivalents de terbutyldicarbonate (PM= 218,25). Le pH est ajusté entre 8 et 9 à l'aide d'une solution de Na₂CO₃ 1N et maintenu constant dans les premières heures de réaction. La cinétique de couplage est contrôlée par chromatographie sur couche mince de silice .

Après évaporation sous vide de l'alcool tertiobutylique , le produit est repris dans 100 ml d'eau. La phase aqueuse est acidifiée à pH 3 par une solution d'HCl 1N. Le Boc-acide aminé est extrait à l'aide d'acétate d'éthyle ( 2 x 100 ml) . La phase organique est lavée à l'eau distillée, séchée sur Na₂SO₄ anhydre, filtrée puis concentrée à l'aide de l'évaporateur rotatif . Le résidu huileux est additionné de 4 ml d'hexane . La cristallisation du Boc-acide aminé est favorisée par refroidissement en chambre froide . Les cristaux blancs sont récupérés sur verre fritté de porosité 4 , lavés à l'hexane et séchés en dessicateur sur P₂O₅.

Rendement : 16 à 18%.

### 1.3. Purification et caractérisation.

### 1.3.1. Purification.

Des recristallisations successives ont permis d'augmenter la pureté (augmentation du point de fusion) . La purification a conduit à une baisse de rendement d'au maximum 2% pour l'acide aminé hexadécanoïque et d'au maximum 1% pour l'acide aminé protégé.

### 1.3.2. Caractérisation.

### A.Point de fusion :

| Produit | Point de fusion obtenu |
|---|---|
| Acide 2-bromohexadécanoïque | 56°C |
| Acide amino-2 hexadécanoïque | 144°C |
| Acide tert-butyloxycarbonyl amino-2 hexadécanoïque | 85°C. |

### B. Chromatographie sur couche mince de silice.

Les solutions ( 10 à 20µl d'une solution à 1 mg/ml) sont déposées sur couche mince de silice (Merck gel de silice 60 sans indicateur de fluorescence).

L'acide 2-bromohexadécanoïque est mis en solution dans l'éthanol , l'acide aminé 2-hexadécanoïque dans l'acide acétique bouillant, et le tert-butyloxycarbonylamino-2-hexadécanoïque dans le dichlorométhane .

Choix du solvant de migration.

Les différents systèmes choisis sont :
Système A: butanol/acétate d'éthyl/acide acétique/eau dans les proportions volume/volume : 1/1/1/1.
Système B: acétate d'éthyl/pyridine/acide acétique/eau dans les proportions v/v: 5/5/1/3.
Système C: chloroforme/méthanol/acide acétique dans les proportions v/v : 10/1/o,1.

### Révélation .

Après migration, dans le système de solvants appropriés , les couches minces sont séchées 15 minutes à 120°C avant d'être révélées après aspersion d'un réactif de révélation.
- Le réactif à la ninhydrine spécifique des fonctions amines primaires permet la révélation de l'acide aminé hexadécanoïque non protégé mais aussi du Boc acide aminé, l'aspersion d'acide acétique à 20% suivie d'un séchage à 120°C permettant le déplacement du groupement Boc .

L'aspersion à l'aide d'un réactif composé de 20g de (NH₄)₂SO₄, de 3 ml de H₂SO₄ et 100 ml d'H₂O permet la révélation simultanée des trois produits. Pour cette technique , le séchage de la chromatographie sur couche mince après aspersion, est réalisé à l'aide d'un épiradiateur ( résistance en porcelaine avec rayonnement infra-rouge ).

Résultat :

| Produit | Solvants | Rf |
|---|---|---|
| Acide 2-bromohexadécanoïque | Système A | 0,5 |
| | Système B | 1 |
| | Système C | 1 |
| Acide amino-2 hexadécanoïque | Système A | 0,82 |
| | Système B | 0,94 |
| | Système C | 0 |
| Acide tert-butyloxycarbonyl amino-2 hexadécanoïque | Système A | 1 |
| | Système B | 1 |
| | Système C | 0,67 |

### C. La spectrométrie de masse (PDMS BIO-ION 20).

Spectre de l'acide tert-butyloxycarbonyl amino-2 hexadécanoïque.

| MM (g) | (M-H)⁻ | Fragments: |
|---|---|---|
| MM théorique | 370,557 | |
| MM expérimentale | 370,8 | 270 |

L'ion moléculaire expérimental et l'ion moléculaire théorique ont une masse identique . L'ion moléculaire se fragmente ; il y a perte du groupement Boc ( pic à 270) . Le pic 296,6 représente l'ion de masse 270 additionné du groupement CN (nitrocellulose).

### 2. Synthèse du 3-β-(2'carboxyméthoxy) cholest-5-ène.

### 2.1. Synthèse du tosylate de cholestéryle.

- Formule brute : C₃₄H₅₃SO₃,
- Masse moléculaire : 540,83,
- Mode opératoire :

Après dissolution de 25,86 mmoles de cholestérol (10g) dans un minimum de pyridine ( 5 à 10 ml), on ajoute un excès de chlorure de tosyle (10g, 52,6 mmoles). Après agitation pendant 12 heures, la pyridine est éliminée par évaporation à sec. Le résidu est solubilisé dans 20 ml d'acétone à chaud (la température est maintenue inférieure à 55°C pour éviter la formation d'une huile ) . On filtre . Le tosylate de cholestérol est obtenu par cristallisation à partir de l'éluat. Les cristaux blancs obtenus sont lavés à l'acétone froid et séchés en dessicateur sur P₂O₅.

Rendement : 82 à 85%.

### 2.2. Synthèse du 3β-(2'hydroxyéthoxy)-cholest-5-ène.

- Formule brute : C₂₉H₅₀O₂.
- Masse moléculaire : 430,71.
- Mode opératoire:

A 17,5 mmoles de tosylate de cholestéryle (10g) dissous dans 120 ml de dioxanne, sont additionnés 30 ml d'éthylèneglycol (480 mmoles) . Le milieu réactionnel est chauffé à reflux pendant 4 heures à 120°C. Après refroidissement , il est repris dans 150 ml d'eau distillée . Le dérivé alcool formé est extrait à l'éther diéthylique ( 3 x 200 ml) . La phase éthérée est lavée successivement par du NaHCO₃ 5% ( 2 x 200 ml) et de l'eau distillée (2 x 200 ml). Après séchage sur Na₂SO₄ anhydre, la solution éthérée est concentrée jusqu'à obtention d'une huile. Après addition de 4 ml d'hexane, la cristallisation est amorcée par frottement et refroidissement en chambre froide (4°C) . Les cristaux blancs sont récupérés sur verre fritté de porosité 4 et lavés à l'hexane avant d'être séchés en dessicateur sur P₂O₅.
Rendement : 32 à 34 %.

### 2.3 . Synthèse du 3β-(2'-carboxyméthoxy)-cholest-5-ène.

- Formule brute : C₂₉H₄₈P₃,
- Masse moléculaire : 444,69.
- Mode opératoire :

On réalise préalablement la solution oxydante : celle-ci comprend 2,67 g d'anhydride chromique, 2,3 ml de H₂SO₄ concentré, le volume étant porté à 10 ml avec de l'eau distillée . Le milieu oxydant est ajouté goutte à goutte à 4,66 mmoles (2g) de 3 β-(2'-hydroxyéthoxy)-cholest-5-ène dissous dans 50 ml d'acétone . L'évolution de la réaction est contrôlée par chromatographie sur couche mince. La réaction achevée, le milieu réactionnel est repris dans 250 ml d'eau distillée . Le dérivé acide est extrait à l'acétate d'éthyle ( 3 x 200 ml). La phase organique est lavée à l'eau distillée ( 2 x 200 ml), séchée sur Na₂SO₄ anhydre et concentrée jusqu'à obtention d'une huile . L'huile est additionnée de 4 ml d'éther de pétrole . On favorise la cristallisation du dérivé acide par refroidissement en chambre froide (4°C) . Les cristaux blancs sont récupérés sur verre fritté de porosité 4, lavés à l'aide d'éther de pétrole et séchés en dessicateur sur P₂O₅.

Rendement: 29 à 31 %.

### 2.4.Purification et caractérisation.

### 2.4.1. Purification.

Le p-toluènesulfonate de cholestéryle est purifié par recristallisations successives dans l'acétone. Le β-(2'-hydroxyéthoxy)-cholest-5-ène et le dérivé acide ont tous deux été purifiés par chromatographie sur couche épaisse de silice.

### A. Chromatographie sur couche épaisse de silice.

Les dépôts sont effectués sur couche épaisse de silice (Merck gel de silice 60 PF ₂₅₄ avec indicateur de fluorescence), les tâches étant révélées par des ultraviolets.

Une solution contenant 0,250 mg de produit est déposée sur la plaque de silice, les produits ont tous deux été dissous dans le dichlorométhane .

| Produit | Solvant | Rf |
|---|---|---|
| 3β-(2'-hydroxyéthoxy)-cholest-5-ène | Ether de pétrole/Ether éthylique Proportions volume/volume:1/1 | 0,48 |
| 3β-(2'-carboxyméthoxy)-cholest-5-ène | Ether de pétrole/Ether éthylique/méthanol Proportions v/v:10/10/3 | 0,52 |

Les deux produits ont été extraits de la silice avec du méthanol . On observe pour chacun des produits une perte équivalente à environ 30% de la quantité déposée .

### 2.4.2. Caractérisation.

### A. Point de fusion.

| Produit | Point de fusion (littérature) | Point de fusion |
|---|---|---|
| Paratoluène sulfonate de cholestéryle | 131,5°C-132,5°C | 128°C |
| 3β-(2'-hydroxyéthoxy) | 102°C-104°C | 99°C |
| 3β-(2'-carboxyméthoxy) cholest-5-ène. | 160°C-161°C | 157°C |

### B .Chromatographie sur couche mince.

Les dépôts (10 à 20 µl) d'une solution 1 mg/ml sont effectués sur couche mince de silice avec indicateur de fluorescence (Merck Kieselgel 60F₂₅₄).

La mise en solution des différents produits est effectuée dans le dichlorométhane.

Après migration, dans le système de solvant approprié, les couches minces sont séchées à l'air avant d'être révélées soit par ultra-violet , soit après aspersion avec HClO₄ ( 20%) et séchage à l'étuve (120°C pendant 20 minutes).

Résultat :
Différents systèmes de solvants.
Système A: Ether éthylique/Ether de pétrole dans les proportions volume/volume : 1/1.
Système B: Ether éthylique/Ether de pétrole dans les proportions v/v : 1/2.
Système C: Ether de pétrole/Ether éthylique/Méthanol dans les proportions v/v : 5/5/1.
Système D: Ether de pétrole/Ether éthylique/Méthanol dans les proportions v/v: 10/10/3.
Système E: Ether de pétrole/Ether éthylique/Méthanol dans les proportions v/v: 5/5/2.
Système F : Ether éthylique.

| Produit | Système de solvant | Rf |
|---|---|---|
| Cholestérol | Système A | 0,54 |
| | Système B | 0,3 |
| | Système F | 0,95 |
| Paratoluène sulfonate de cholestéryle | Système A | 0,85 |
| | Système B | 0,62 |
| | Système F | 1 |
| 3β-(2'-hydroxyéthoxy)-cholest-5-ène | Système A | 0,41 |
| | Système B | 0,24 |
| | Système F | 0,9 |
| 3β-(2'-carboxyméthoxy)-cholest-5-ène | Système A | 0 |
| | Système B | 0 |
| | Système C | 0,1 |
| | Système D | 0,42 |
| | Système E | 0,78 |
| | Système F Effet de trainée: RfO--> | 0,5 |

### C. Spectrométrie de masse (PDMS).

Analyse du 3β-(2'-carboxyméthoxy)-cholest-5-ène.

| | |
|---|---|
| MM (g) | (M-H)- |
| MM théorique | 443,69 |
| MM expérimentale | 443,1 |

L'ion moléculaire expérimental et l'ion moléculaire théorique ont une masse identique.

### D. Résonance magnétique nucléaire du C13.

L'analyse du spectre du 3β-(2'-carboxyméthoxy)-cholest-5-ène a été effectuée par comparaison avec le spectre RMN C13 du cholestérol.

La mise en solution du cholestérol et du 3β-(2'-carboxyméthoxy)-cholest-5-ène a été réalisée dans le chloroforme deutéré.

Spectre du cholestérol.

| Pics | d(ppm) obtenu | attribution | d(ppm)théorique |
|---|---|---|---|
| 1 | 140,7606 | C5 ou C6 | fonction alcène: d(ppm) de 100)145 |
| 2 | 121,7064 | C5 ou C6 | |
| 3 | 78,5715 | CDCl₃ | |
| 4 | 76,9981 | CDCl₃ | |
| 5 | 75,4010 | CDCl₃ | |
| 1' à 22' | 71 à 11 | | fonctions alcanes. |

Spectre du 3 β-(2'carboxyméthoxy)-choloest-5-ène.

| Pics | d(ppm) obtenu | attribution | d(ppm)théorique |
|---|---|---|---|
| 1 | 172,2923 | C2' | fonction acide:d(ppm) de 160 à 185 |
| 2 | 139,8394 | C5 ou C6 | fonction alcène: d(ppm) de 100 à 145 |
| 3 | 122,5233 | C5 ou C6 | fonction alcène fonction éther:d(ppm) de 45 à 80 |
| 5 | 79,2943 | Cl' | + léger déplacement |
| 6 | 78,5903 | CDCl₃ | |
| 7 | 77,0089 | CDCl₃ | |
| 8 | 75,4146 | CDCl₃ | |
| 9 à 31 | 65 à 11 | | fonctions alcanes. |

### E. Analyse élémentaire .

L'analyse élémentaire du 3β-(2'-carboxyméthoxy)-cholest-5-ène a fourni les résultats suivants:

| | Théorique | Obtenu |
|---|---|---|
| % de carbone | 78,3 | 76,25 |
| % d'hydrogène | 10,9 | 10,9 |
| % d'oxygène | 10,8 | 12,5 |

### EXEMPLE 2 - Synthèse des lipopeptides.

### I.Méthode de couplage de l'acide 2-amino hexadécanoïque.

Le choix des séquences construites sous forme lipopeptidique s'est porté sur la région 312-327 de la gp120 du virus VIH-1 LAV -BRU afin d'étudier la réponse T cytotoxique . On a effectué 3 préparations avec cette séquence.

La synthèse a été réalisée en phase solide (MERRIFIELD R.B., 1963, J. Am. Chem. Soc. 85, 2149-2210) .

Tous les lipopeptides ont été synthétisés sur une résine benzhydrylamine (chargée à 0,72 millimole/gramme). Dans tous les cas , le premier acide aminé greffé est l'acide BOC amino-2 hexadécanoïque ( 2 équivalents).Ceci a permis d'obtenir des constructions où l'acide aminé C-terminal est l'acide amino-2 hexadécanoïque sous forme amide afin d'éviter la présence d'une charge à proximité de la chaîne aliphatique hydrophobe. Après acétylation par l'anhydride acétique en milieu basique, afin de bloquer les sites réactifs libres, on a effectué le clivage du BOC N-terminal puis le couplage du premier acide aminé de la séquence.

Toutes ces étapes ont été réalisées manuellement, ce qui a permis d'effectuer des contrôles très précis des couplages de l'acide aminé pseudolipidique et du premier acide aminé sur celui-ci.

L'activateur de couplage est l'hexafluorophosphate de benzotriazolyl-N-oxytriadiméthylaminophosphonium (BOP) en présence d'hydroxybenzotriazole (HOBt) et de diisopropyléthylamine (DIEA). Grâce à cette méthode de couplage très performante, le rendement de ces deux réactions de couplage a toujours été supérieur à 99,5% malgré le fort encombrement stérique dû à l'acide BOC amino-2 hexadécanoïque.

La suite des synthèses s'est effectuée de façon classique en automatique jusqu'au dernier acide aminé. A ce stade la peptidyl-résine a été divisée en 3 lots, traités manuellement :
- 1 lot a été conservé tel quel;
- 1 lot a été greffé par l'acide BOC amino-2 hexadécanoïque.
Le couplage manuel (au BOP) de ce dernier a été suivi d'un clivage du BOC N-terminal et d'une acétylation de toutes les fonctions amines ainsi démasquées . Cela a permis d'éviter la présence d'une charge à proximité de la chaîne aliphatique hydrophobe de l'acide aminé pseudolipidique ,
- 1 lot a été greffé par la diBOC, Nα,ε-lysine. Le couplage manuel ( au BOP) de cette dernière a été suivi d'un clivage des deux BOC et du couplage manuel de l'acide palmitique ( au BOP). Nous avons ainsi obtenu des peptides possédant une dipalmitoyl-lysine en position N-terminale.

Ces couplages effectués manuellement ont fait l'objet d'un contrôle étroit qui a révélé des rendements toujours supérieurs à 99,5%. Ces résultats confirment l'intérêt de l'utilisation du BOP comme agent activant en synthèse peptidique, surtout pour coupler les acides aminés pseudolipidiques ou pour effectuer un couplage sur ces derniers. Les lipopeptides synthétisés ont ensuite été clivés de leur support . Les lipopeptides dérivés de la séquence 312-327 ont été clivés par traitement à l'acide fluorhydrique anhydre. Les rendements de coupure sont assez faibles, compris entre 40 et 70%.

Il existe au moins deux explications à ces valeurs :
1) le clivage d'un peptide greffé sur une résine benzhydrylamine n'est jamais complet dans les conditions usuelles de coupure;
2) la présence de l'acide amino-2 hexadécanoïque, directement en contact avec la résine, a certainement amplifié ce phénomène.

Après deux lavages (TFA-éther), l'identité des lipopeptides a été contrôlée en analyse d'acide aminé après hydrolyse acide totale et , pour certains, en spectrométrie de masse PDMS. Leur homogénéité a été vérifiée en chromatographie sur couche minche de silice et CLHP en phase inverse analytique.

### II. Méthodes de couplage du pimélautide et du triméxautide.

### 1) Méthode de couplage du pimélautide ( ou du triméxautide ) à l'extrémité N-terminale d'un peptide par le moyen d'un maillon succinyle.

Cette méthode s'applique à la fixation du pimélautide ( ou du triméxautide ) non protégé sur un peptide construit en phase solide , encore fixé sur la résine, et aux chaînes latérales protégées .

Le triméxautide et le pimélautide (Rhône Poulenc) présentent tous deux , deux fonctions carboxyliques libres, et une fonction amine primaire libre . La création d'une liaison amide prédéterminée entre le peptide et le piumélautide ( ou le triméxautide), ne peut se faire que par utilisation du pimélautide ( ou de triméxautide) comme partenaire aminé .

La succinylation du peptide sur la résine permet d'en faire le partenaire carboxylique.

### DEPROTECTION

Le groupement terbutyloxycarbonyle, qui protège temporairement l'extrémité N-terminale du peptide sur la résine, est clivé par l'action d'une solution d'acide trifluoroacétique à 40% dans le dichlorométhane , pendant 20 minutes sous agitation.

La résine est lavée par :
- deux fois 20 ml de dichlorométhane,
- deux fois 20 ml de diisopropyléthylamine à 5% dans le dichlorométhane ,
- deux fois 20 ml de diméthylformamide (pendant 3 minutes pour chaque lavage ).

### SUCCINYLATION.

Elle est réalisée en effectuant trois fois le couplage par mise en présence de la résine de la solution de succinylation avec :
- un quintuple excès d'anhydride succinique ( solution à 5% dans la N-méthylpyrrolidone ).
- de la diisopropyléthylamine en quantité stoechiométrique par rapport aux amines de la résine (pendant 20 minutes sous agitation ).

### ACTIVATION.

L'activation du carboxyle maintenant présent sur la résine est réalisée de la façon suivante :

la résine est soumise à l'action de la solution activante ( pendant 15 minutes à température ambiante, et sous agitation ):
- B.O.P. ( hexafluorophosphate de benzotriazolyloxy trisdiméthylamino phosphonium) : 3 excès par rapport aux carboxyles,
- H.O.B.T. ( hydroxybenzotriazole) : 3 excès par rapport aux carboxyles,
- diisopropyléthylamine : 7 excès par rapport aux carboxyles en solution dans la N-méthylpyrrolidone .

### LAVAGE:

La solution est lavée par :
- 3 fois 30 ml de diméthylformamide,
- 3 fois 30 ml de dichlorométhane.

### COUPLAGE:

La résine est soumise à l'action de la solution de couplage :
- pimélautide ( ou triméxautide): 3 excès par rapport à l'ester activé d'hydroxybenzotriazole,
- diisopropyléthylamine : 3 excès par rapport à l'ester,
- diméthylsulfoxyde 10%
- N - méthylpyrrolidone 90 %: quantité suffisante pour dissoudre le pimélautide ( ou le triméxautide ).

La solution saturée est environ à 4% de pimélautide ou de triméxautide après sonication et passage 2 minutes à 50°C.

### 2)Synthèse en phase solide du V3GP120, 312-327 succinyl.

### a) N-protection du pimélautide ( ou du triméxautide) par le groupement tert-butyloxycarbonyle.

500 micromoles de pimélautide ( ou de triméxautide ) sont dissous dans 10 ml d'une solution tampon carbonate 0,1 M à pH 9,5.

On ajoute 10 ml d'une solution de pyrocarbonate de diterbutyle à 100 mmoles/l.

Un pH compris entre 9 et 10 est maintenu pendant 100 heures par ajoût de carbonate disodique.

Le milieu réactionnel est ensuite dilué par 10 ml d'eau et 10 ml d'éther diéthylique, et la phase aqueuse lavée est acidifiée à pH 2,5 par l'hydrogénosulfate de potassium.

Une extraction par 100 ml de dichlorométhane, suivi d'une évaporation du solvant à l'évaporateur rotatif conduit à la cristallisation du Boc-pimélautide ( ou Boc-triméxautide).

L'incorporation du Boc-pimélautide ( ou du Boc-triméxautide ) en synthèse peptidique en phase solide génère deux isomères de position.

### b) CLIVAGE ET PURIFICATION.

Le clivage du peptide en fin de synthèse est réalisé par l'acide fluorhydrique anhydre.

Le peptide est alors purifié par gel-filtration et HPLC préparative en phase inverse de type C₄.

La figure 1 représente le spectre à 235 nm de l'HPLC préparative , obtenu sur 20 mg de lipopeptide dissous dans HCOOH.

Le lipopeptide obtenu est alors analysé par hydrolyse acide totale, par chromatographie analytique HPLC C₄ et en spectrographie de masse.

La figure 2 représente le spectre de masse. On observe un pic distinc à 2422,2 ce qui correspond à la masse du lipopeptide.

Les figures 3a et 3b représentent respectivement la chromatographie analytique HPLC C₄ du lipopeptide et du témoin sans lipopeptide.

Les conditions de la chromatographie sont les suivantes :
solvant (A) : trifluoroacétate à 0,5%o. gradiant :
solvant B: acétonitrile 0,75%. trifluoroacétate à 0,5%o. gradiant de 10% à 80% en 120 mn. mesure à une longueur d'onde de 215 nm.
Lors de l'hydrolyse acide totale, l'acide diaminopimélique (Dap) présent dans le pimélautide et le triméxautide constitue un bon marqueur du couplage.

| Résultats de l'hydrolyse acide totale | | | | |
|---|---|---|---|---|
| Amino-acides | nanomoles | mesuré | théorique | mesuré/ théorique |
| Thr | 3.2500 | 0.97 | 1 | 0.97/1 |
| Glu | 7.1000 | 2.11 | 2 | 1.06/1 |
| Pro | 3.1800 | 0.95 | 1 | 0.95/1 |
| Gly | 10.3500 | 3.08 | 3 | 1.03/1 |
| Arg | 6.6900 | 1.99 | 2 | 1.00/1 |
| Val | 3.3900 | 1.01 | 1 | 1.01/1 |
| Ile | 9.5800 | 2.85 | 3 | 0.95/1 |
| Phe | 3.3500 | 1.00 | 1 | 1.00/1 |
| Lys | 3.5200 | 1.05 | 1 | 1.05/1 |
| Arg | 9.9200 | 2.95 | 3 | 0.98/1 |
| Dap | 3.500 | 1.05 | 1 | 1.05/1 |

### EXEMPLE 3

### Immunisation à l'encontre du peptide NP 147-158.

Les immunisations des souris sont effectuées comme suit :

### Immunisations:

Les souris ont été injectées avec les préparations de lipopeptides par voie intrapéritonéalle ( i.p.) ou par voie sous-cutanée (S.C.), avec ou sans adjuvant .

Il faut au moins deux injections ( espacées de 8 à 30 jours) pour obtenir des CTL.

Chaque injection contient 5 x 10⁻⁸ mole de lipopeptide.

### Détection des CTL

8 à 21 jours après la dernière injection, la rate des souris immunisées a été prélevée, les splénocytes de ces souris ont été cultivés in vitro à raison de 5 x 10⁶ splénocytes/2 ml de milieu de culture classique (DMEM + 10% Fes + pyruvate + acides aminés non essentiels + β-2-Mercaptoéthanol) contenant 5µM du peptide correspondant à celui impliqué dans la construction lipopeptidique.

A partir du 5ème jour de culture in vitro, l'activité des CTL peut être détectée par le test classique de relarguage de ⁵¹Cr. ( Martinon et al, J. Immunol., 142; 3489-3494, 1989).

L'activité des CTL est testée à l'encontre de cellules cibles syngéniques en présence du peptide (NP 147-158 R⁻, P3CSS Pep.NP, ou L1-Pep.NP) ou à l'encontre de cellules cibles infectées par le virus influenza A.

Les résultats obtenus sont résumés dans le tableau I. La première partie du tableau concerne des résultats déjà obtenus, avec le virus influenza total, la protéine NP 147-158 R du virus influenza et le lipopeptide P3 CSS-PEPNP, qui est composé du peptide NP 147-158 couplée à la tripalmitoyl S-glycérylcystéinyle-séryle-sérine (DERES et al. précédemment cité ) .

La seconde partie du tableau concerne d'une part les essais d'immunisation effectués avec des liposomes contenant le peptide NP 147-158, et avec des lipopeptides L1, L2 et L3. Ces lipopeptides sont des molécules contenant respectivement une partie peptidique (NP 147-158) et une partie lipidique. Les lipopeptides L1, L2 et L3 ont donc les formules suivantes :

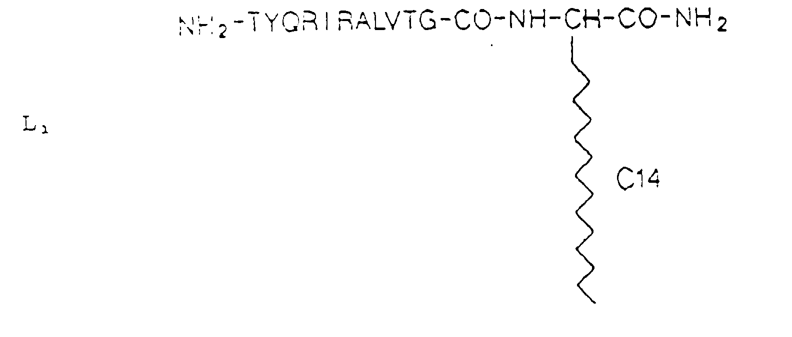

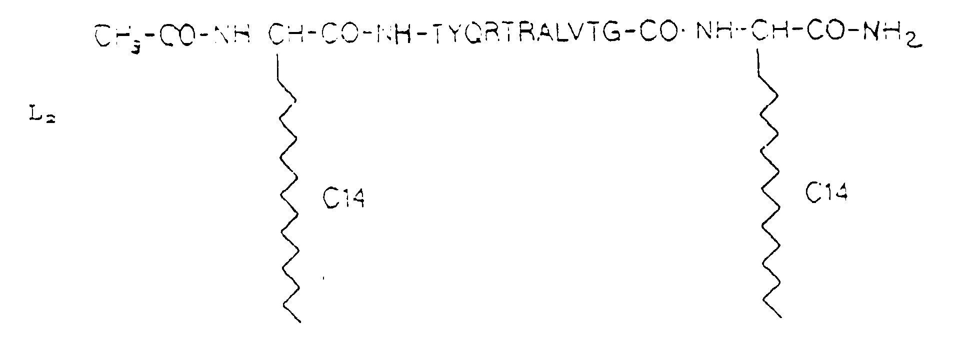

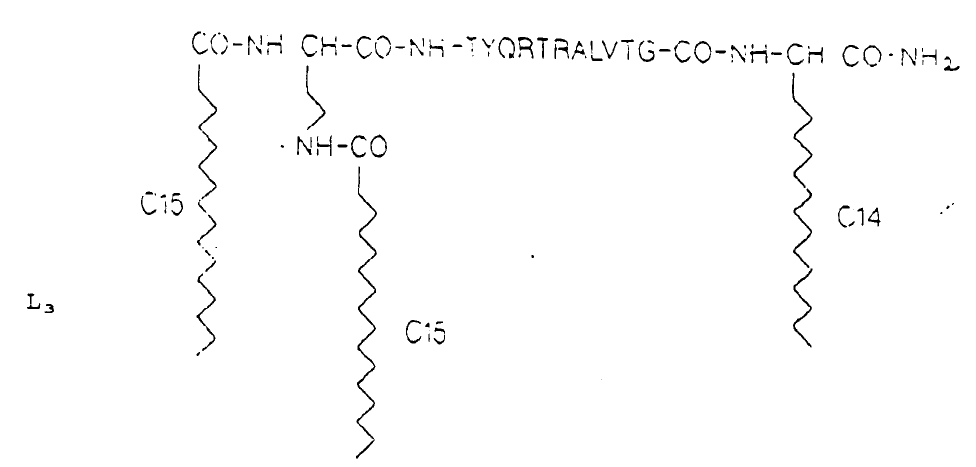

Les activités cytolytiques après 5 jours, 12 jours et plus de 21 jours montrent que l'on obtient une très forte activité pour le lipopeptide L1 comparé aux autres essais effectués.

### EXEMPLE 4

### Immunisation par CB₁, CB₂ et CB₃ à l'encontre du peptide ENV 312-327.

Ce peptide est un fragment de protéine, codé par le gène env du virus HIV.

Les protocoles expérimentaux sont identiques à ceux décrits dans l'exemple 3.

Le tableau II résume les résultats obtenus.

Dans ce tableau, CB₁, CB₂ et CB₃ correspondent à des lipopeptides formés à partir du peptide issu de la protéine ENV et d'un lipide.

Les formules de CB₁, CB₂ et CB₃ sont les suivantes :

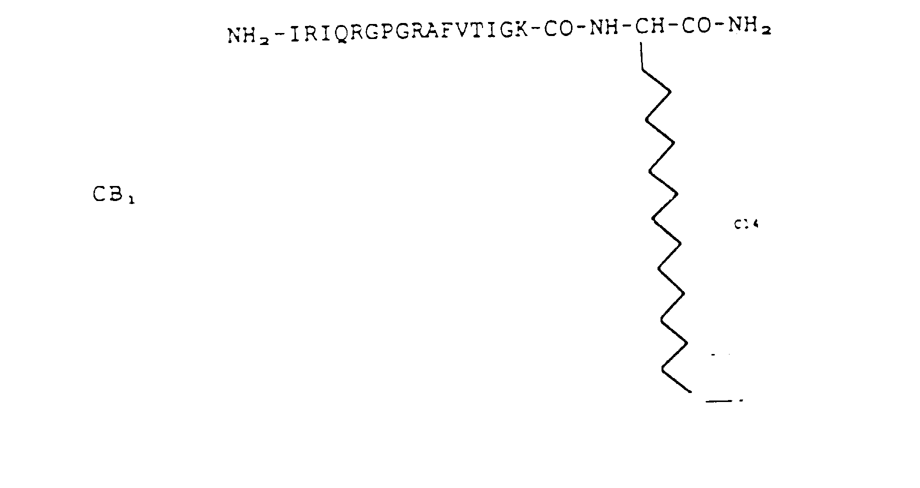

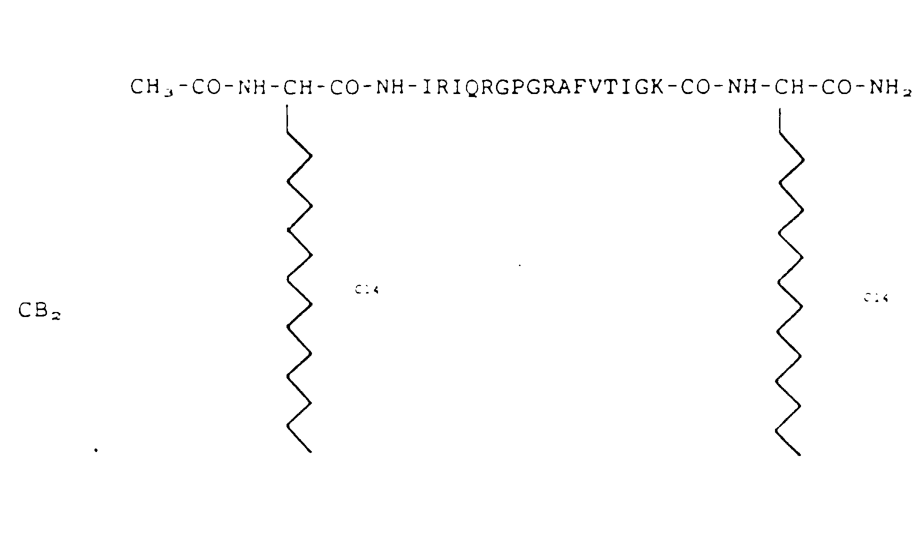

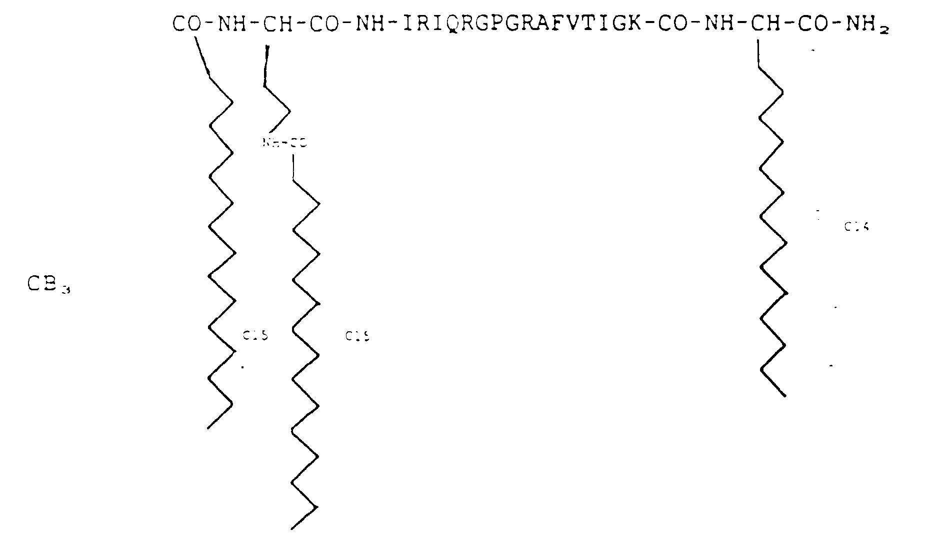

Les résultats du tableau II montrent une activité importante pour l'un des lipopeptides (CB₁).

### EXEMPLE 5:

### Immunisation à l'encontre du peptide ENV 302-335.

Ce peptide est le fragment 302 à 335 de la protéine ENV du virus HIV.

Les protocoles expérimentaux sont identiques à ceux décrits dans l'exemple 3.

Les résultats sont figurés dans le tableau III.

Les formules des lipopeptides CB₆, CB₇ et CB₈ sont les suivantes:

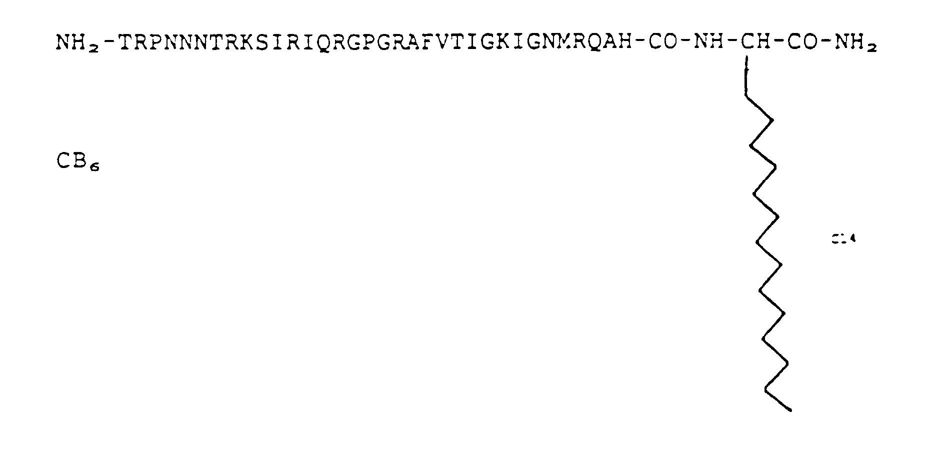

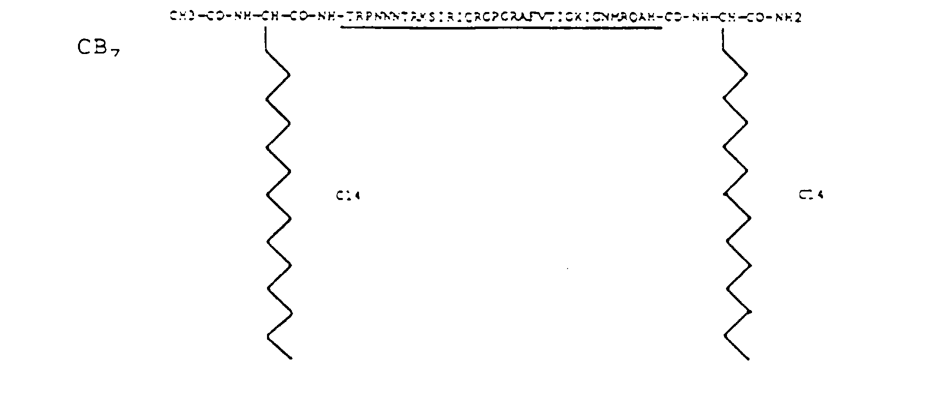

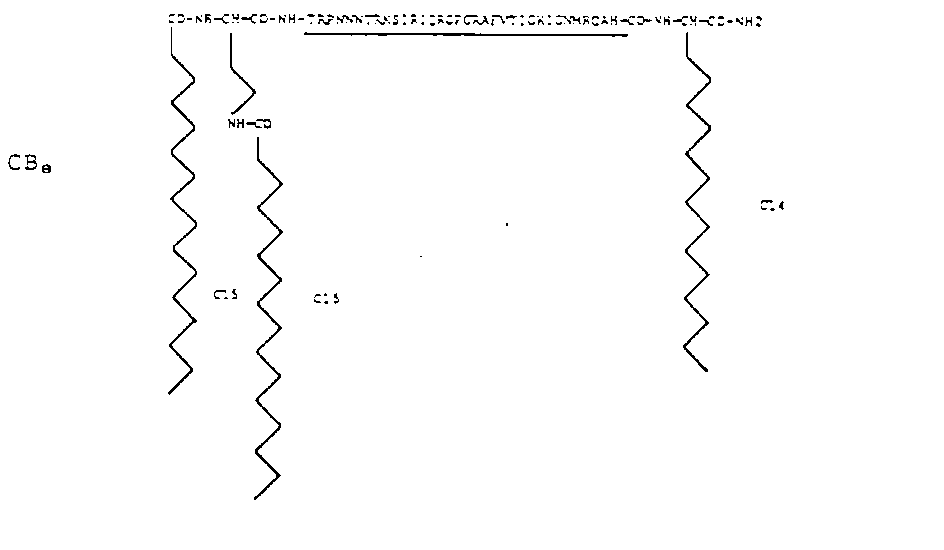

On remarque dans le tableau III que les deux lipopeptides CB₆ et CB₇ montrent des activités cytolytiques très supérieures au témoin.

### EXEMPLE 6:

### Immunisation par CB₁, CB₄,CB₅ CB₁₇, CB₁₉,CB₂₁ et CB₂₅ à l'encontre du peptide ENV 312-327.

Les protocoles expérimentaux sont sensiblement identiques à ceux décrits dans l'exemple 3.

Le tableau IV reprend les résultats obtenus.

La formule de CB1 est indiquée dans l'exemple 4.

Les formules de CB₄,CB₅ CB₁₇, CB₁₉, CB₂₁ et CB₂₅ sont les suivantes : 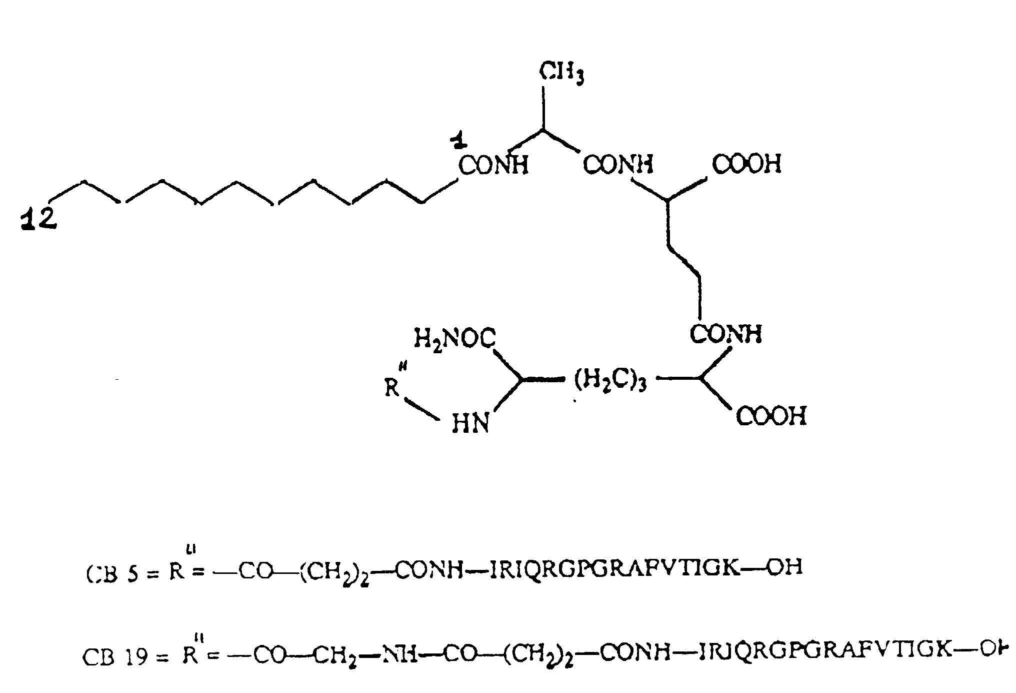 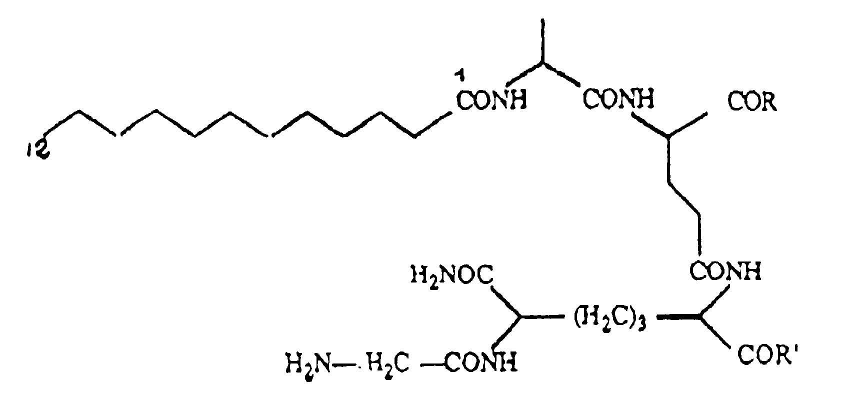 CB 21 Mélange de
- A:: R = OH
R' = NH-IRIQRGPGRAFVTIGK-OH
- B:: R = NH-IRIQRGPGRAFVTIGK-OH
R' = OH

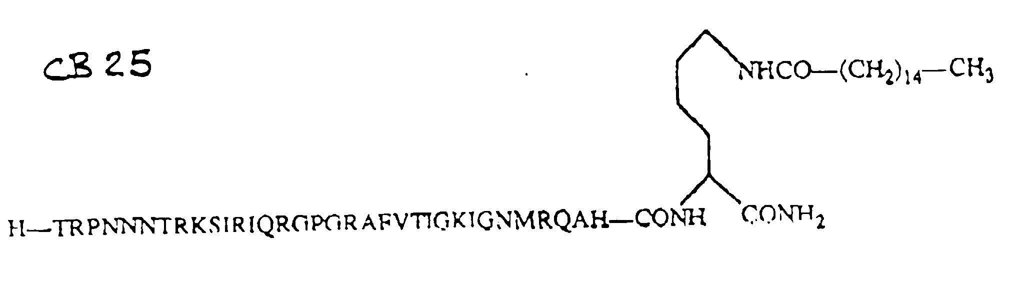

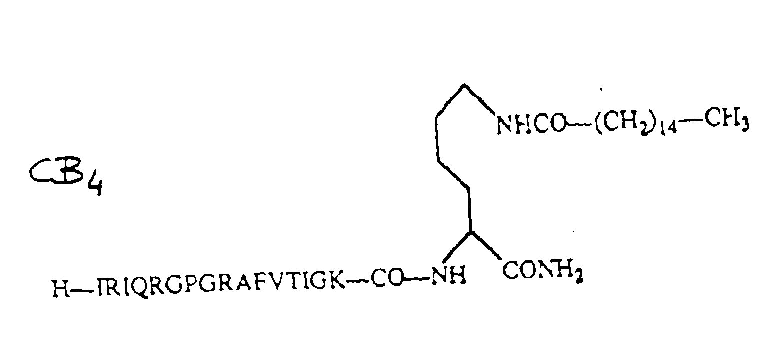

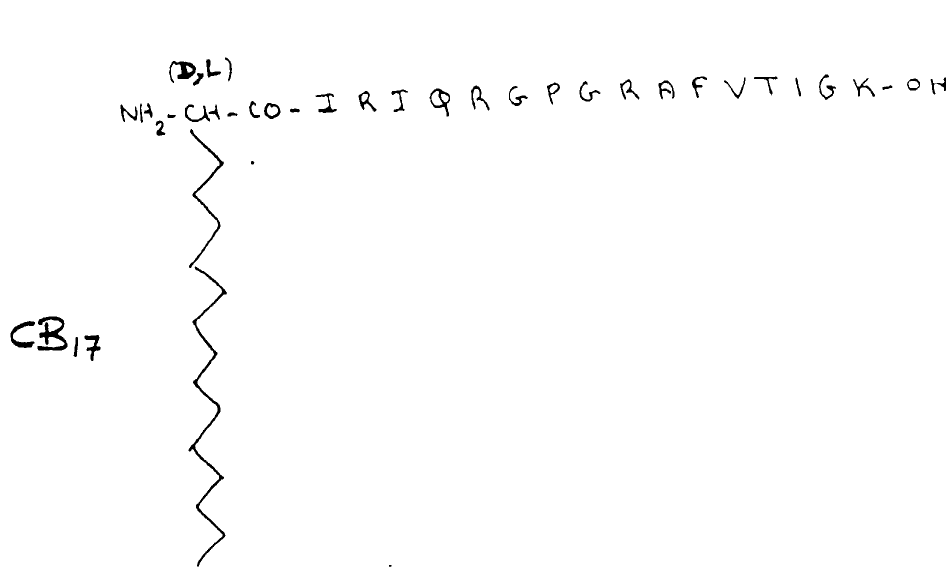

L'extrémité C-terminale de la séquence 312-327 de la protéine GP 120 de HIV-1 (LAV-BRU) est sous forme carboxylique . Un acide a amino hexadécanoïque racémique a été fixé sur la région N-terminale . La fonction N-terminale est sous forme amine.

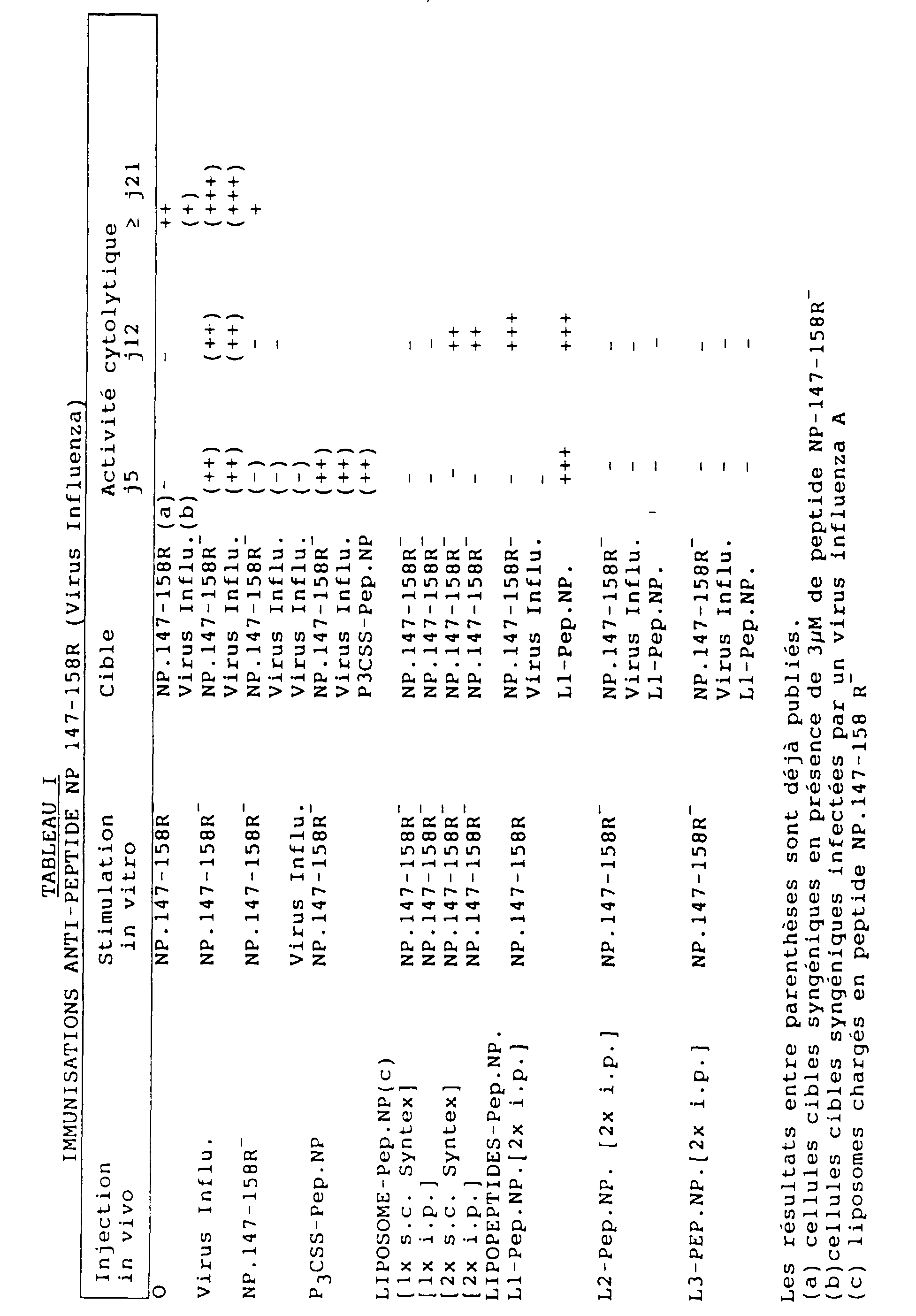

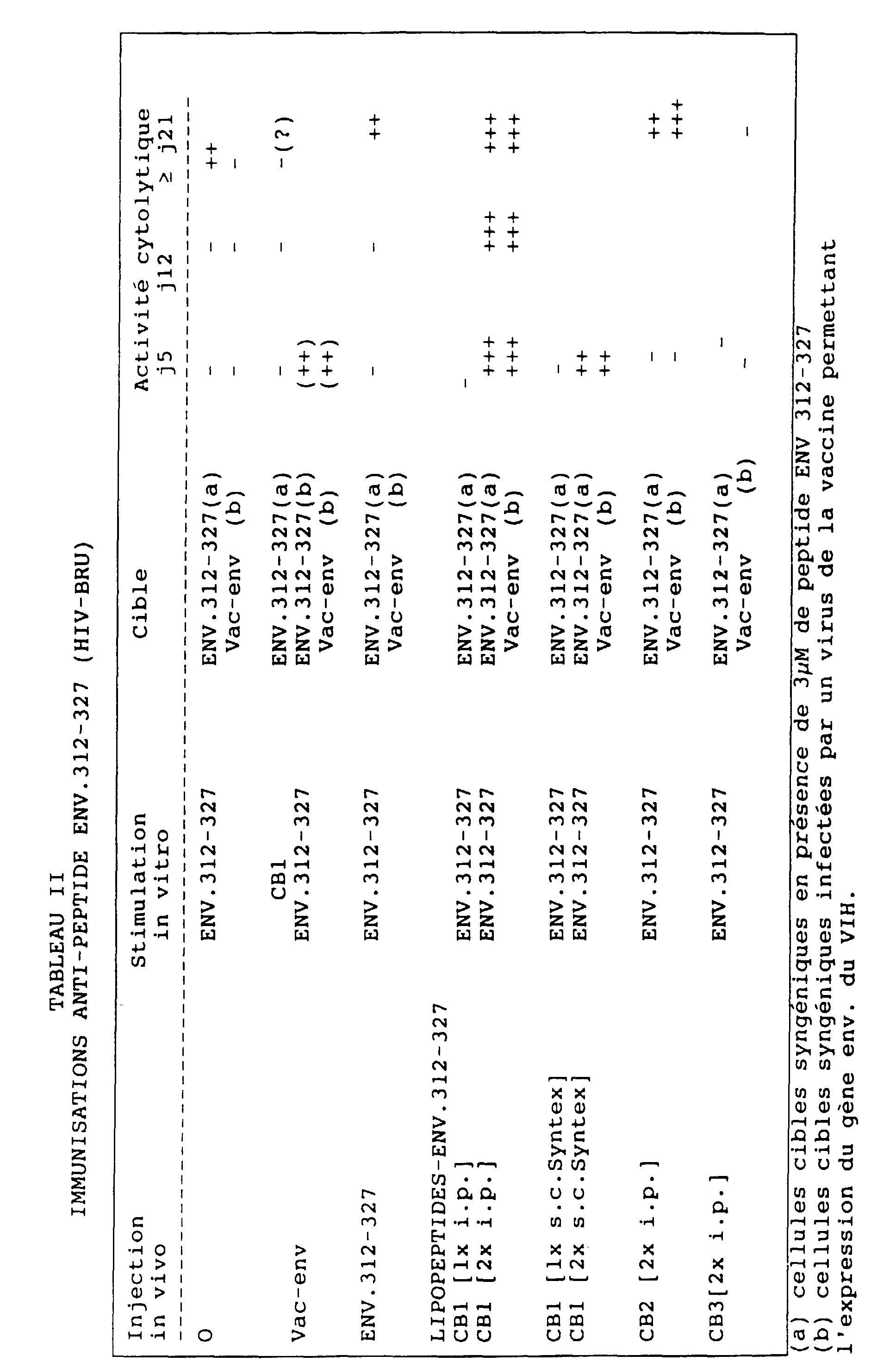

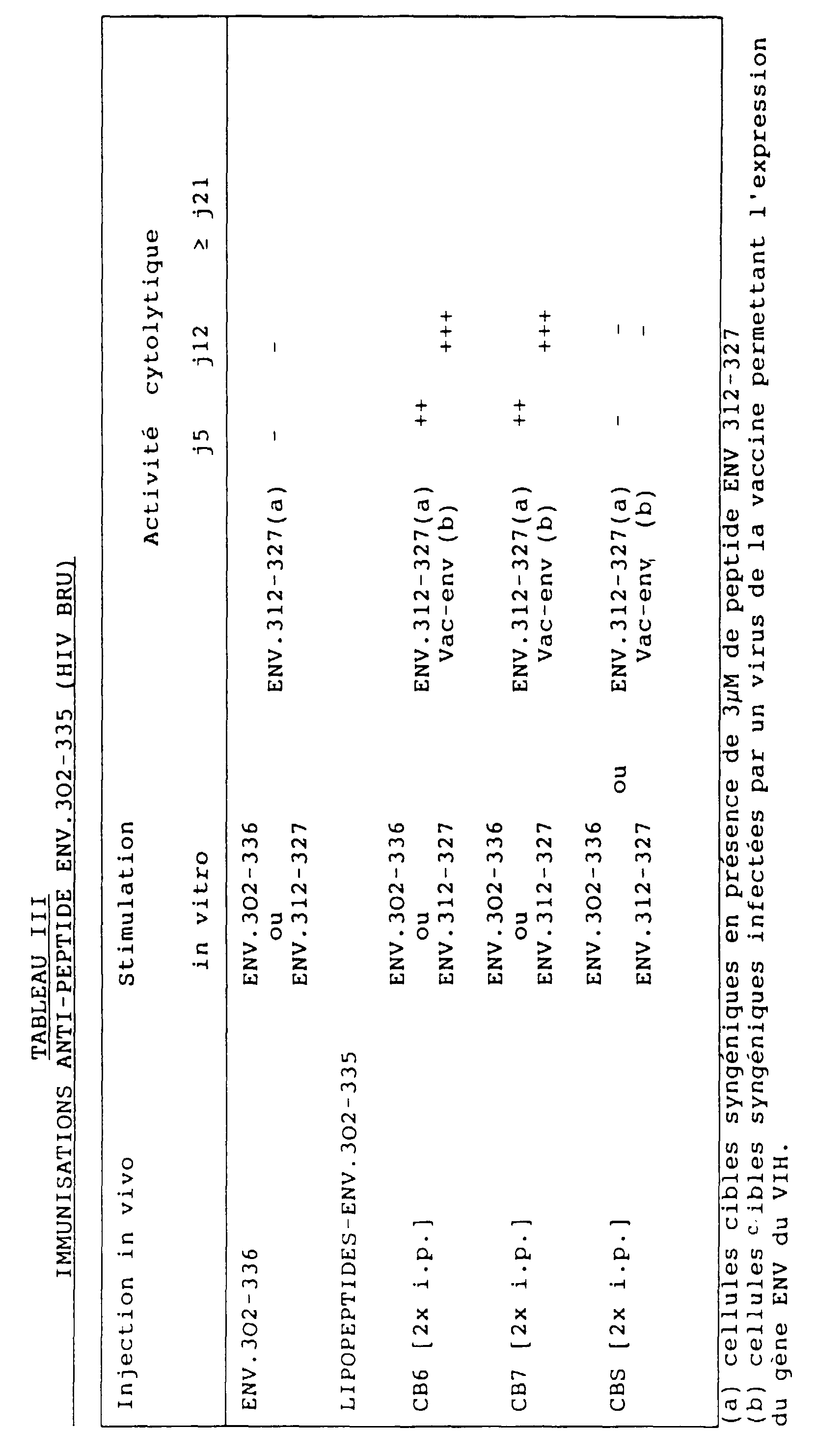

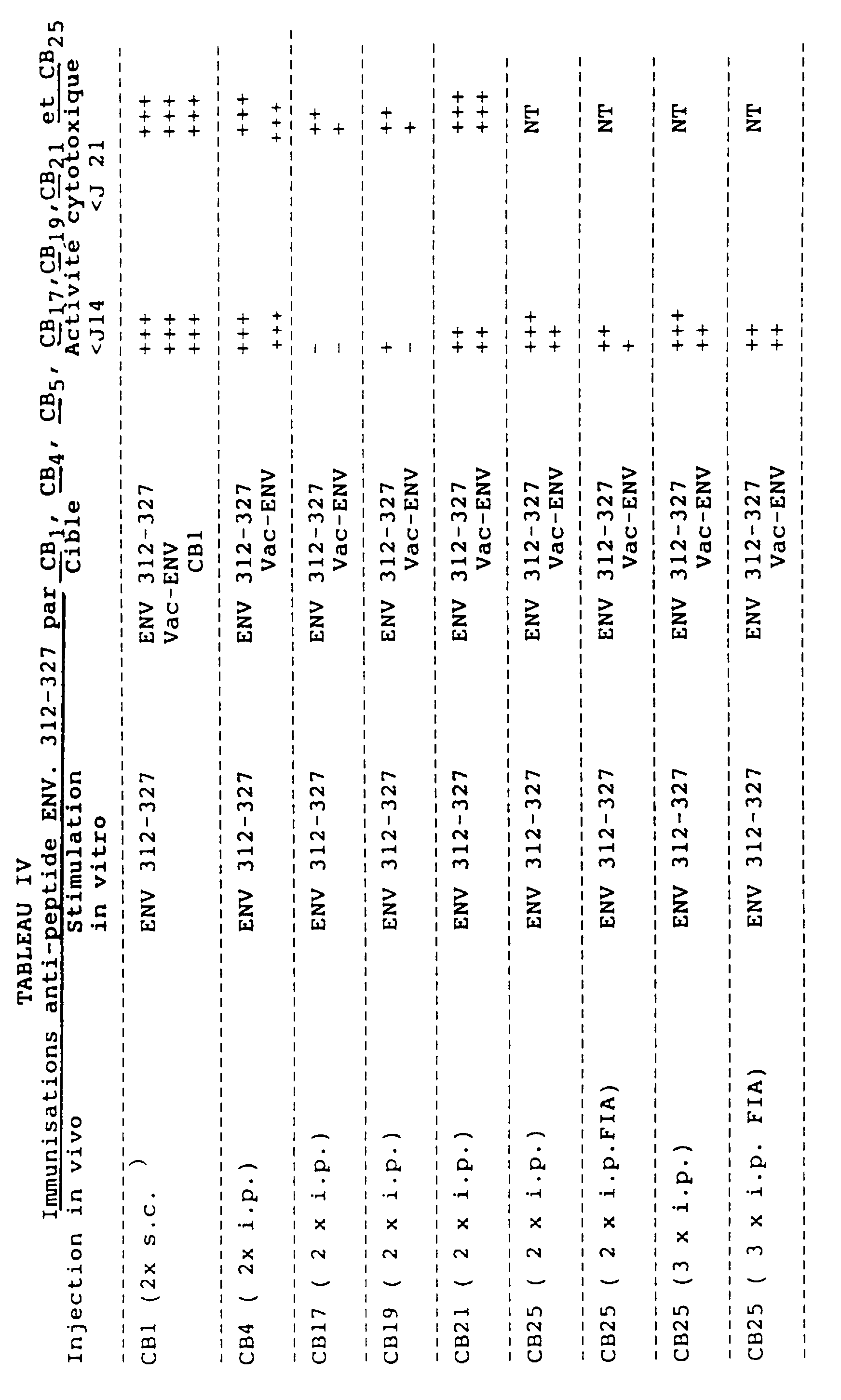

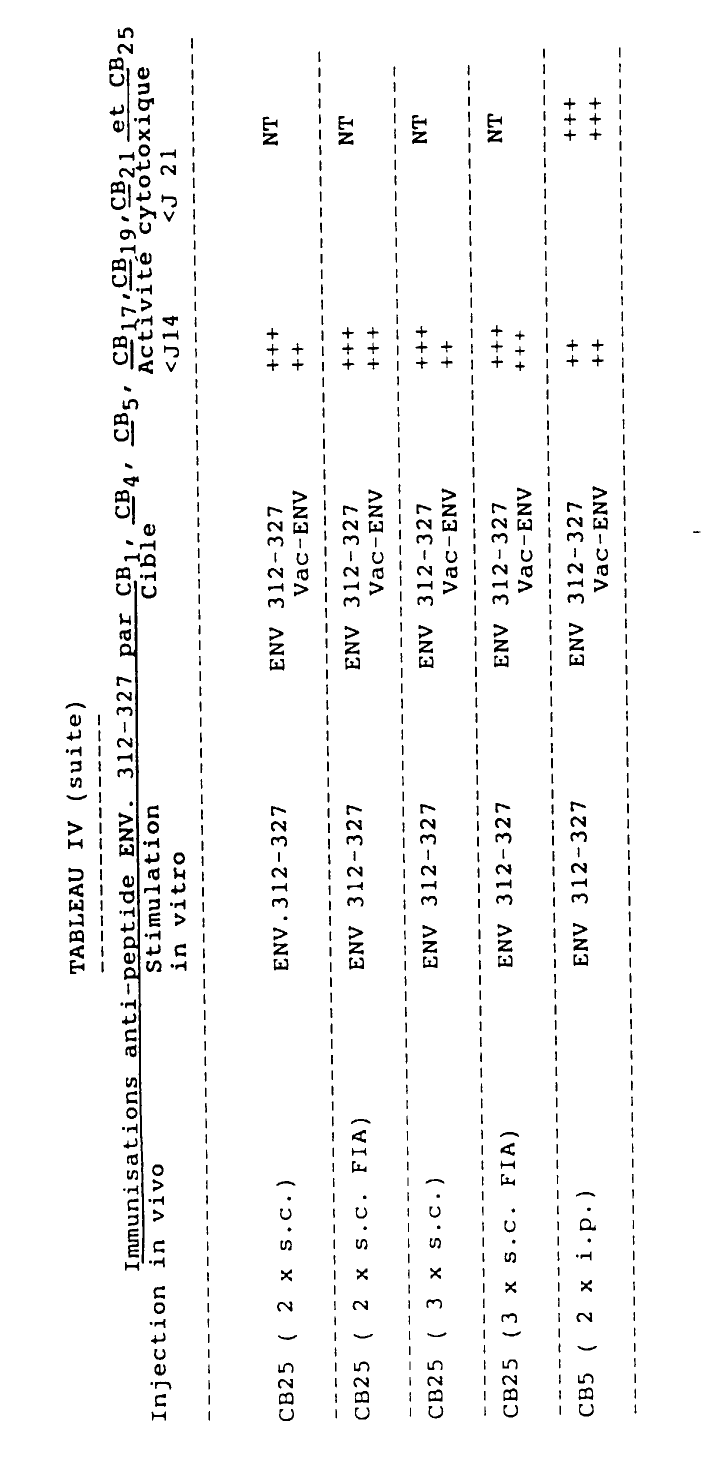

## Revendications

1. Utilisation d'un lipopeptide comprenant une partie peptidique ayant entre 10 et 40 acides aminés environ, et préférentiellement entre 10 et 20 acides aminés environ, et comportant au moins un déterminant antigénique induisant spécifiquement des lymphocytes T-cytotoxiques, ledit lipopeptide comprenant également une ou plusieurs chaînes dérivées de la N-ε-palmitoyllysine couplées sur des fonctions αNH₂ ou εNH₂ desdits acides aminés pour la fabrication d'un médicament pour l'immunisation du corps humain ou animal à l'encontre d'agents pathogènes.

2. Utilisation selon la revendication 1, caractérisée en ce que la chaîne dérivée est la N,N'-dipalmitoyl-lysine.

3. Utilisation selon la revendication 1, caractérisée en ce que la chaîne dérivée est la N-palmitoyl-lysine.

4. Utilisation selon la revendication 1, caractérisé en ce que la chaîne dérivée est la N-ε-palmitoyl-lysylamide.

5. Utilisation selon l'une des revendications 1 à 4 caractérisée en ce que les agents pathogènes sont des virus.

6. Utilisation selon l'une des revendications 1 à 5 dans laquelle les virus sont HIV1 et HIV2.

7. Lipopeptide susceptible d'être utilisé selon la revendication 6 comprenant un fragment d'une protéine des virus HIV1 ou HIV2, ayant entre 10 et 40 acides aminés environ, et préférentiellement entre 10 et 20 acides aminés environ, et comportant au moins un déterminant antigénique induisant spécifiquement des lymphocytes T-cytotoxiques, ledit lipopeptide comprenant également une ou plusieurs chaînes dérivées de la N-ε-palmitoyl-lysine, couplées sur des fonctions αNH₂ ou εNH₂ desdits acides aminés.

8. Lipopeptide selon la revendication 7 caractérisé en ce que la chaîne dérivée est la N-palmitoyl-lysine.

9. Lipopeptide selon la revendication 7 caractérisé en ce que la chaîne dérivée est la N-ε-palmitoyl-lysylamide.

10. Lipopeptide selon la revendication 7 caractérisé en ce que la chaîne dérivée est la N'N'-dipalmitoyl-lysine.

11. Lipopeptide selon l'une des revendications 7 à 10, caractérisé en ce que la protéine est celle codée par le gène ENV.

12. Lipopeptide selon l'une des revendications 7 à 11, caractérisé en ce qu'il comprend le fragment 312-327 ou le fragment 302-306 de la protéine codée par le gène ENV.

## Claims

1. Use of a lipopeptide comprising a peptide part having between about 10 and 40 amino acids and preferably between about 10 and 20 amino acids and comprising at least one antigenic determinant inducing specifically cytotoxic T lymphocytes, said lipopeptide also comprising one or more chains derived from N-ε-palmitoyl-lysine coupled on the αNH₂ or εNH₂ functional groups of said amino acids for the manufacture of a medicinal product for immunizing the human or animal body against pathogenic agents.

2. Use according to claim 1, characterized in that the derived chain is N,N'-dipalmitoyl-lysine.

3. Use according to claim 1, characterized in that the derived chain is N-palmitoyl-lysine.

4. Use according to claim 1, characterized in that the derived chain is N-ε-palmitoyl-lysylamide.

5. Use according to one of claims 1 to 4, characterized in that the pathogenic agents are viruses.

6. Use according to one of claims 1 to 5 wherein vinises are HIV1 and HIV2 viruses.

7. Lipopeptide liable to be used according to claim 6 comprising a protein moiety of HIV1 or HIV2 viruses having between about 10 and 40 amino acids and preferably about between 10 and 20 amino acids and comprising at least one antigenic determinant inducing specifically cytotoxic T lymphocytes, said lipopeptide also comprising one or more chains derived from N-ε-palmitoyl-lysine coupled on the αNH₂ or εNH₂ functional groups of said amino acids.

8. Lipopeptide according to claim 7, characterized in that the derived chain is N-palmitoyl-lysine.

9. Lipopeptide according to claim 7, characterized in that the derived chain is N-ε-palmitoyl-lysylamide.

10. Lipopeptide according to claim 7, characterized in that the derived chain is N,N'-dipalmitoyl-lysine.

11. Lipopeptide according to one of claims 7 to 10, characterized in that the protein is encoded by the ENV gene.

12. Lipopeptide according to one of claims 7 to 11, characterized in that it comprises the 312-327 fragment or 302-306 fragment of the protein encoded by the ENV gene.

## Patentansprüche

1. Verwendung eines Lipopeptids, das einen Peptidteil mit ungefähr zwischen 10 und 40 Aminosäuren und vorzugsweise ungefähr zwischen 10 und 20 Aminosäuren umfaßt und das wenigstens eine antigene Determinante trägt, die cytotoxische T-Lymphocyten spezifisch induziert, wobei das Lipopeptid außerdem eine oder mehrere von N-ε-Palmitoyllysin abgeleitete Ketten umfaßt, die an α-NH₂- oder ε-NH₂-Funktionen der Aminosäuren gekuppelt sind, zur Herstellung eines Medikaments zur Immunisierung des menschlichen oder tierischen Körpers gegenüber Krankheitserregern.

2. Verwendung gemäß Anspruch 1, dadurch gekennzeichnet, daß es sich bei der abgeleiteten Kette um N,N'-Dipalmitoyllysin handelt.

3. Verwendung gemäß Anspruch 1, dadurch gekennzeichnet, daß es sich bei der abgeleiteten Kette um N-Palmitoyllysin handelt.

4. Verwendung gemäß Anspruch 1, dadurch gekennzeichnet, daß es sich bei der abgeleiteten Kette um N-ε-Palmitoyllysylamid handelt.

5. Verwendung gemäß einem der Ansprüche 1 bis 4, dadurch gekennzeichnet, daß die Krankheitserreger Viren sind.

6. Verwendung gemäß einem der Ansprüche 1 bis 5, wobei es sich bei den Viren um HIV1 und HIV2 handelt.

7. Lipopeptid, das gemäß Anspruch 6 verwendet werden kann und ein Fragment eines Proteins der Viren HIV1 oder HIV2 mit ungefähr zwischen 10 und 40 Aminosäuren und vorzugsweise ungefähr zwischen 10 und 20 Aminosäuren umfaßt und das wenigstens eine antigene Determinante trägt, die cytotoxische T-Lymphocyten spezifisch induziert, wobei das Lipoprotein außerdem eine oder mehrere von N-ε-Palmitoyllysin abgeleitete Ketten umfaßt, die an α-NH₂- oder ε-NH₂-Funktionen der Aminosäuren gekuppelt sind.

8. Lipopeptid gemäß Anspruch 7, dadurch gekennzeichnet, daß es sich bei der abgeleiteten Kette um N-Palmitoyllysin handelt.

9. Lipopeptid gemäß Anspruch 7, dadurch gekennzeichnet, daß es sich bei der abgeleiteten Kette um N-ε-Palmitoyllysylamid handelt.

10. Lipopeptid gemäß Anspruch 7, dadurch gekennzeichnet, daß es sich bei der abgeleiteten Kette um N,N'-Dipalmitoyllysin handelt.

11. Lipopeptid gemäß einem der Ansprüche 7 bis 10, dadurch gekennzeichnet, daß es sich bei dem Protein um das von dem Gen env codierte handelt.

12. Lipopeptid gemäß einem der Ansprüche 7 bis 11, dadurch gekennzeichnet, daß es das Fragment 312-327 oder das Fragment 302-306 des von dem Gen env codierten Proteins umfaßt.
